# EUROPÄISCHE PATENTSCHRIFT

(11) **EP 3 351 180 B1**
(45) Veröffentlichungstag und Bekanntmachung des Hinweises auf die Patenterteilung: **25.09.2019**
(21) Anmeldenummer: 17185687.5
(22) Anmeldetag: 10.08.2017
(51) Int. Cl.: A61B 6/10

(54) **ÜBERWACHUNGSVERFAHREN UND ÜBERWACHUNGSSYSTEM**
MONITORING METHOD AND MONITORING SYSTEM
PROCÉDÉ DE SURVEILLANCE ET SYSTÈME DE SURVEILLANCE

(43) Veröffentlichungstag der Anmeldung: 25.07.2018
(73) Patentinhaber: Siemens Healthcare GmbH, 91052 Erlangen (DE)
(72) Erfinder: Pickert, Nils, 91056 Erlangen (DE); Weingarten, Markus, 96049 Bamberg (DE)

(56) Entgegenhaltungen:
- WO-A1-2015/132069
- WO-A1-2015/162101
- US-A1- 2011 037 840
- US-A1- 2011 317 815
- US-A1- 2016 206 274
- US-B1- 6 435 717

## Beschreibung

Die Erfindung betrifft ein Verfahren zur Überwachung der Strahlenexposition von medizinischem Personal gemäß dem Patentanspruch 1 sowie ein Überwachungssystem zur Durchführung eines derartigen Verfahrens gemäß dem Patentanspruch 13.

Für medizinisches Personal, welches im Umfeld von Röntgenstrahlung aussendenden medizinischen Bildgebungsgeräten tätig ist, ist es schwierig, genau einzuschätzen, wo genau sich direkte Röntgenstrahlung oder auch Streustrahlung befindet. Aus diesem Grund kommt es häufig vor, dass sich unbeabsichtigt Körperteile, vor allem Arme und Hände von medizinischem Personal im Strahlengang oder in von Streustrahlung betroffenen Zonen befinden. Dies tritt besonders häufig im Umfeld von interventionellen und chirurgischen Eingriffen unter Röntgenstrahlung auf, wo die Hände von Ärzten während der Operation besonders nah am Patienten/Untersuchungsobjekt positioniert sind. Durch die direkte Röntgenstrahlung entsteht ein nicht unerhebliches gesundheitliches Risiko für die betroffenen Personen, vor allem wenn dies über einen längeren Zeitraum geschieht, auch Streustrahlung z.B. durch Streuung an Geräten oder dem Patienten stellt ein Risiko dar. Außerdem wird durch die Körperteile im direkten Strahlengang zusätzliche Streustrahlung erzeugt und auch die Bildgebung negativ beeinflusst. Insgesamt ist es wünschenswert, dass Bewusstsein für die Gefahr zu steigern und insgesamt die Gefahr der unerwünschten Strahlenexposition zu reduzieren.

Aus der Patentanmeldung WO 2015/132069 A1 ist ein Überwachungsverfahren während einer Strahlenexposition offenbart, bei welchem z.B. durch Näherungssensoren detektiert werden kann, ob medizinisches Personal sich zu nah an dem bestrahlten Bereich befindet.

Es ist Aufgabe der vorliegenden Erfindung, ein Verfahren zur Überwachung von medizinischem Personal bereitzustellen, welches einen zuverlässigen Hinweis auf eine unerwünschte Strahlenexposition ermöglicht; des Weiteren ist es Aufgabe der Erfindung, ein für die Durchführung des Verfahrens geeignetes Überwachungssystem bereitzustellen.

Die Aufgabe wird erfindungsgemäß gelöst durch ein Verfahren zur Überwachung der Strahlenexposition von medizinischem Personal gemäß dem Patentanspruch 1 und von einem Überwachungssystem gemäß dem Patentanspruch 13. Vorteilhafte Ausgestaltungen der Erfindung sind jeweils Gegenstand der zugehörigen Unteransprüche.

Das erfindungsgemäße Verfahren zur Überwachung der Strahlenexposition von medizinischem Personal während einer Röntgenuntersuchung eines Untersuchungsobjekts mit einem Röntgengerät umfasst die folgenden Schritte: Aktivierung einer Überwachungseinheit, wobei die Überwachungseinheit ein erstes dreidimensionales Volumen, welches einen von dem direkten Röntgenstrahl bestrahlbaren und/oder bestrahlten Bereich umfasst, kontinuierlich nach Objekten abscannt, wobei die Aktivierung der Überwachungseinheit getriggert wird durch die Aktivierung einer einen Röntgenstrahl aussendenden Röntgenquelle, im Falle einer Detektion eines Objekts automatische Auswertung, ob es sich bei dem Objekt um einen menschlichen Körperteil handelt, welcher nicht dem Untersuchungsobjekt entspricht, und Ausgabe eines Signals oder einer Anzeige, wenn ein menschlicher Körperteil innerhalb des ersten dreidimensionalen Volumens ermittelt wird, welcher nicht dem Untersuchungsobjekt entspricht. Durch das Verfahren wird während der Applikation von Röntgenstrahlung kontinuierlich überwacht, ob sich ein menschlicher Körperteil von medizinischem Personal im Strahlengang befindet und sobald ein solcher Körperteil entdeckt wird, erfolgt umgehend ein Hinweis auf die Gefahr. Dadurch wird dem medizinischen Personal die Gefahr sofort bewusst gemacht und die betroffene Person kann sofort ihren Körperteil aus der Gefahr entfernen. Durch das Verfahren wird das Gefahrenbewusstsein verstärkt und das gesundheitliche Risiko für medizinisches Personal deutlich gesenkt, da die Dauer der Strahlenexposition gesenkt werden kann.

Nach einer Ausführung der Erfindung wird das erste dreidimensionale Volumen abhängig von dem Strahlengang des Röntgenstrahls insbesondere automatisch vorgegeben. Hierfür können z.B. Informationen über den Strahlengang (z.B. Kollimation usw.) an die Überwachungseinheit weitergegeben und verwendet werden. In vorteilhafter Weise für eine besonders zeitnahe Überwachung werden Informationen über den von dem Röntgenstrahl bestrahlten Bereich kontinuierlich erneuert und das erste dreidimensionale Volumen entsprechend angepasst.

Nach einer weiteren Ausgestaltung der Erfindung wird die Überwachungseinheit von zumindest einer 3D-Kamera oder einem Infrarotgerät oder einer Terahertzkamera gebildet. Derartige Vorrichtungen eigenen sich besonders gut für eine umfassende Überwachung von 3-dimensionalen Raumabschnitten. Es können auch zusätzlich oder alternativ andere Überwachungsgeräte verwendet werden.

Nach einer weiteren Ausgestaltung der Erfindung wird die Auswertung von einem Computervisionsverfahren durchgeführt. Hierfür können z.B. ein Objekterkennungs- oder Objektklassifikationsverfahren verwendet werden. Zweckmäßigerweise werden für die automatische Auswertung neuronale Netzwerke verwendet.

Nach einer weiteren Ausgestaltung der Erfindung wird im Fall einer Ermittlung eines menschlichen Körperteils innerhalb des ersten dreidimensionalen Volumens, welcher nicht dem Untersuchungsobjekt entspricht, eine Anpassung der Kollimation des Röntgenstrahles der Röntgenquelle durchgeführt, um die Gefahr für die Person, deren Körperteil sich im Strahlengang befindet, zu senken. In vorteilhafter Weise ändert die Kollimation den von dem Röntgenstrahl bestrahlten Bereich derart, dass der menschliche Körperteil nicht mehr oder mit geringerer Dosis bestrahlt wird. So kann z.B. ein sogenannter Fingerfilter automatisch in den Strahlengang geschoben werden, welcher den Körperteil von der Strahlung abschottet.

Nach einer weiteren Ausgestaltung der Erfindung scannt die Überwachungseinheit zusätzlich ein zweites dreidimensionales Volumen, welches einen von Streustrahlung getroffenen Bereich umfasst, nach Objekten ab und es wird automatisch ausgewertet, ob es sich bei dem Objekt um einen menschlichen Körperteil handelt, welcher nicht dem Untersuchungsobjekt entspricht, und es wird ein Signal oder eine Anzeige ausgegeben, wenn ein menschlicher Körperteil innerhalb des zweiten dreidimensionalen Volumens ermittelt wird, welches nicht dem Untersuchungsobjekt entspricht. Auf diese Weise kann das medizinische Personal auch bezüglich der Streustrahlung auf eine Gefahrensituation hingewiesen werden und so das Risiko weiter reduziert werden.

Erfindungsgemäß wird die Aktivierung der Überwachungseinheit getriggert durch die Aktivierung einer einen Röntgenstrahl aussendenden Röntgenquelle. Es kann auch eine sich zumindest über einen längeren Zeitraum als die Röntgenstrahlungsapplikation erstreckende Überwachung vorgesehen sein. Hier kann auch während eines Zeitraums ohne Röntgenstrahlung ein potentiell von Röntgenstrahlung getroffenes Volumen nach Objekten abgescannt und ausgewertet werden. Wird ein Körperteil ermittelt, welches sich bei Aktivierung der Strahlenquelle im Strahlengang befindet, so kann hier ebenfalls eine Signal/Anzeige ausgegeben werden.

Nach einer weiteren Ausgestaltung der Erfindung wird das Signal von einem optischen oder akustischen oder haptischen Signal gebildet. So kann zum Beispiel ein durchgehender lauter Signalton ausgesendet werden oder eine Warnleuchte blinken oder ein Vibrationsalarm ausgelöst werden. Hierbei können entsprechende auffällige Farben und/oder Töne verwendet werden. Es kann auch auf einem Bildschirm eine Anzeige erscheinen, z.B. ein farbig hervorgehobenes Textfeld. In vorteilhafter Weise für eine besonders gute Sichtbarkeit kann auch der ermittelte menschliche Körperteil optisch hervorgehoben und insbesondere beleuchtet werden.

Nach einer weiteren Ausgestaltung der Erfindung erfolgt im Falle einer Detektion eines Objektes zusätzlich eine Auswertung nach vorbestimmten Gegenständen und wird bei Ermittlung eines solchen Gegenstandes im Strahlengang ein weiteres, sich von dem Signal oder der Anzeige für ein Körperteil unterscheidendes Signal oder eine weitere Anzeige ausgegeben. Derartige Gegenstände können z.B. Instrumente, Scheren oder Schläuche oder Halterungen sein, welche durch eine Bestrahlung negativ beeinflusst werden können oder selbst eine Gefahr darstellen.

Zur Durchführung des Verfahrens ist ein Überwachungssystem vorgesehen, welches einem von einer Systemsteuerung angesteuerten Röntgengerät mit einer zur Aussendung eines Röntgenstrahls ausgebildeten Röntgenquelle zugeordnet ist, aufweisend eine von der Systemsteuerung triggerbare Überwachungseinheit, ausgebildet zum kontinuierlichen Abscannen eines ersten dreidimensionalen Volumens nach Objekten, eine Auswerteeinheit, ausgebildet zur Auswertung von mittels der Überwachungseinheit detektierten Objekten dahingehend, ob es sich bei dem Objekt um einen menschlichen Körperteil handelt, welcher nicht dem Untersuchungsobjekt entspricht, und eine Ausgabeeinheit zur Ausgabe eines Signals oder einer Anzeige.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Ansicht eines Röntgengeräts mit einem Überwachungssystem;
- FIG 2: eine Ansicht eines Objekts im vom Röntgenstrahl bestrahlten Volumen;
- FIG 3: ein Ablauf eines erfindungsgemäßen Verfahrens; und
- FIG 4: ein Ablauf des Verfahren wie FIG 3 mit zusätzlichen Schritten.

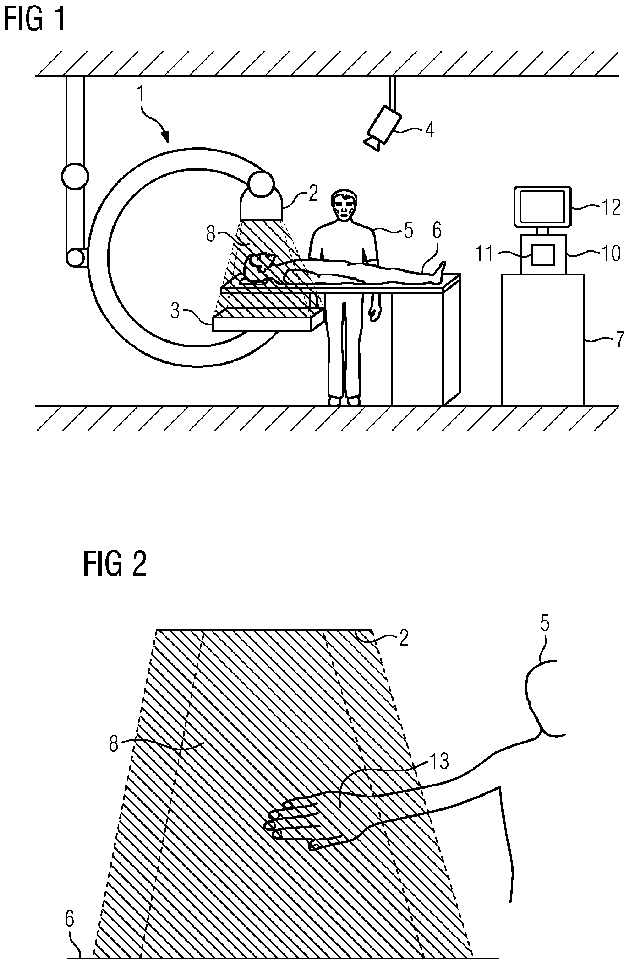

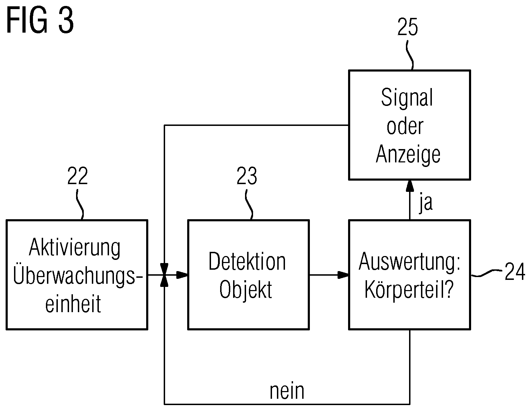

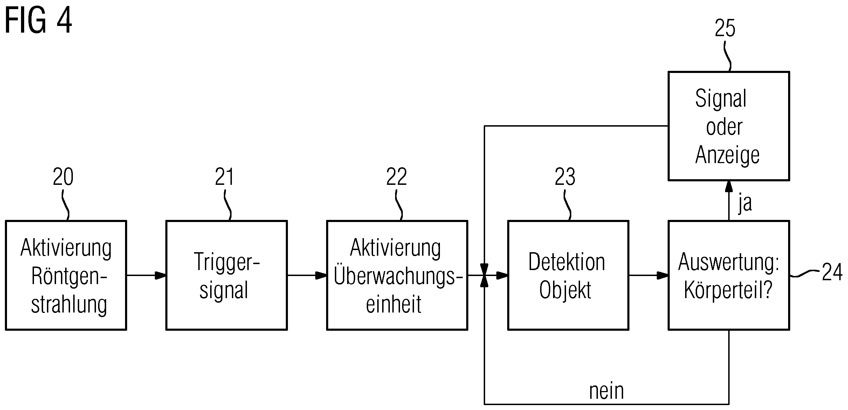

In der FIG 1 ist ein Röntgengerät 1 gezeigt, wobei dem Röntgengerät 1 ein Überwachungssystem gemäß der Erfindung zugeordnet ist. Das Röntgengerät 1 weist einen C-Bogen mit einer an einem Ende angeordneten Röntgenquelle 2 und einem am anderen Ende angeordneten Röntgendetektor 3 auf, wobei die Röntgenquelle zur Aussendung eines Röntgenstrahls ausgebildet ist. Der Röntgenstrahl trifft nach Durchleuchtung eines auf einem Untersuchungstisch angeordneten Untersuchungsobjekt (im Allgemeinen ein Organ eines Patienten 6 auf dem Röntgendetektor 3 auf. Zur Formung des Röntgenstrahls ist ein Kollimator (nicht gezeigt) vorgesehen. Das Röntgengerät wird von einer Systemsteuerung 7 angesteuert. Im Allgemeinen ist medizinisches Personal 5 wie z.B. Ärzte oder Krankenschwestern im Raum mit dem Röntgengerät. Wird z.B. unter Röntgenkontrolle durch das medizinische Personal ein operativer oder interventioneller Eingriff durchgeführt, so besteht die Gefahr, dass ein Körperteil oder mehrere Körperteile, z.B. Hände oder Arme, einer oder mehrerer Personen des medizinischen Personals sich für längere Zeit im Strahlengang des Röntgenstrahls befinden und mit unerwünschter Röntgenstrahlung bestrahlt werden. Dies ist z.B. in der FIG 2 gezeigt, wo sich eine Hand 13 einer Person des medizinischen Personals 5 in einem von dem Röntgenstrahl des Röntgengeräts 1 direkt bestrahlten ersten dreidimensionalen Volumen 8 befindet. Um dies zu verhindern, ist dem Röntgengerät ein Überwachungssystem zugeordnet, welches zumindest eine Überwachungseinheit (gezeigt in Form einer 3D-Kamera 4), eine Steuerungseinheit 10, eine Auswerteeinheit 9 und eine Ausgabeeinheit (gezeigt in Form einer Anzeigeeinheit 12) aufweist. Die Röntgenbestrahlung geschieht z.B. im Rahmen eines interventionellen Eingriffs wie dem Einführen eines Katheters oder dem Einsetzen eines Implantats (z.B. Stent) oder einer endovaskulären Aortenreparatur (EVAR), was unter Röntgenkontrolle durchgeführt werden soll, um eine genaue Positionierung besser überwachen zu können. In der FIG 3 ist der Ablauf des erfindungsgemäßen Verfahrens in mehreren Schritten gezeigt. In einem ersten Schritt 22 wird die Überwachungseinheit aktiviert. Dies kann jederzeit routinemäßig erfolgen, um eine kontinuierliche Überwachung vorzusehen, oder in Verbindung mit einem vorgesehenen oder geplanten Eingriff. Die Aktivierung der Überwachungseinheit steht im Zusammenhang mit einer Aktivierung der Röntgenquelle (siehe FIG 4). Bei der Überwachungseinheit kann es sich z.B. um eine oder mehrere 3D-Kamera(s) 4 handeln, welche in der Lage ist, dreidimensionale Volumina zu überwachen. Es können auch andere Überwachungseinheiten verwendet werden, z.B. Infrarotgeräte oder Terahertzkameras. Das erste dreidimensionale Volumen, das die Überwachungseinheit überwacht, kann zuvor ausgewählt und eingestellt worden sein, zum Beispiel durch einen Nutzer des Systems oder automatisch durch die Systemsteuerung. Das überwachte erste dreidimensionale Volumen 8 umfasst dabei den von Röntgenstrahlung direkt bestrahlten Raumbereich. Es kann auch vorgesehen sein, ein zweites dreidimensionales Volumen zu überwachen, welches von Streustrahlung betroffene Bereiche mit umfasst. Die Überwachungseinheit (also z.B. 3D-Kamera 4) kann zum Beispiel an einer Stelle der Decke oder Wand des Raumes, in dem sich das Röntgengerät befindet, montiert sein, oder auch an dem Patiententisch oder zum Beispiel dem C-Bogen angeordnet sein. Auf diese Weise ist eine besonders gute Sicht der Überwachungseinheit auf das durchstrahlte erste dreidimensionale Volumen möglich.

Ist die Überwachungseinheit aktiviert, so bleibt sie dies idealerweise mindestens solange, bis die Bestrahlung durch die Röntgenquelle beendet wird. Die Überwachungseinheit kann auch kontinuierlich betrieben werden oder z.B. solange sich überhaupt Personen in dem betroffenen Raum aufhalten. Wird während der Überwachung durch die Überwachungseinheit in einem zweiten Schritt 23 ein Objekt in dem Volumen detektiert, so wird daraufhin in einem dritten Schritt 24 - z.B. durch die Auswerteeinheit 9 - das Objekt daraufhin überprüft, ob es sich erstens um einen menschlichen Körperteil handelt und zweitens ob es sich bei dem menschlichen Körperteil um ein nicht dem Untersuchungsobjekt entsprechendes menschliches Körperteil handelt. Eine solches Auswertung kann z.B. mittels eines Algorithmus eines Computervisionsverfahrens durchgeführt werden, also z.B. auf der Basis eines Objekterkennungsverfahrens oder eines Kantenerkennungsverfahrens. Hierfür können zudem lernfähige neuronale Netzwerke verwendet werden, um die Erkennung von menschlichen Körperteilen voranschreitend zu verbessern.

Für den Fall, dass bei der Auswertung ein menschlicher Körperteil detektiert wird, welcher nicht dem Untersuchungsobjekt entspricht, wird in einem vierten Schritt 25 ein Signal erzeugt oder eine Anzeige ausgegeben, um das medizinische Personal zu informieren. So kann z.B. ein Warnton oder ein Warnlicht ausgegeben werden oder es kann z.B. das menschliche Körperteil farbig beleuchtet werden. Es kann auch auf einem Bildschirm oder einem mobilen Gerät eine Textanzeige ausgegeben werden. Die optischen oder akustischen Signale können an bestimmte dafür ausgebildete Vorrichtungen versendet werden, z.B. an den Beeper des entsprechenden Arztes oder an einen im Raum installierten Projektor. Das Signal kann auch von einem haptischen Signal gebildet werden. Insgesamt wird das medizinische Personal unverzüglich auf die Gefahr aufmerksam gemacht, so dass der betroffene menschliche Körperteil schnell aus der Gefahrenzone entfernt werden kann.

Zusätzlich zu dem Signal oder der Anzeige kann auch vorgesehen sein, den Kollimator des Röntgengeräts derart anzusteuern, dass der menschliche Körperteil von der direkten Röntgenstrahlung abgeschirmt ist oder die Dosis zumindest teilweise reduziert wird. Dafür kann z.B. von der Systemsteuerung des Röntgengeräts angesteuert ein Fingerfilter in den Strahlengang des Röntgenstrahls geschoben werden. In dem Fall, dass die Dosis durch einen Filter reduziert (nicht komplett abgeschirmt) wird, kann für die teilabgeschirmte Bildregion eine angepasste Bildverstärkung durchgeführt werden, um eine über das gesamte aufgenommene Bild konstante Helligkeit zu erzielen. Auf diese Weise ist auch weiterhin für die teilabgeschirmte Bildregion eine qualitativ akzeptable Röntgenbildgebung möglich ist.

Das erste dreidimensionale Volumen 8 wird abhängig von dem Strahlengang des Röntgenstrahls insbesondere automatisch vorgegeben. Hierfür können Informationen über den Strahlengang (z.B. die Kollimation usw.) durch die Systemsteuerung des Röntgengeräts an die Steuerungseinheit der Überwachungseinheit weitergegeben und verwendet werden. In vorteilhafter Weise für eine besonders zeitnahe Überwachung werden Informationen über den von dem Röntgenstrahl bestrahlten Bereich kontinuierlich erneuert und das erste dreidimensionale Volumen 8 entsprechend kontinuierlich angepasst.

Zusätzlich zu der Überwachung der menschlichen Körperteile kann auch im Falle einer Detektion eines Objektes zusätzlich eine Auswertung nach vorbestimmten Gegenständen stattfinden. Hier kann dann wird bei Ermittlung eines solchen Gegenstandes im Strahlengang ein weiteres, sich von dem Signal oder der Anzeige für ein Körperteil unterscheidendes Signal oder eine weitere Anzeige ausgegeben werden. Derartige Gegenstände können z.B. Instrumente, Scheren oder Schläuche oder Halterungen sein, welche durch eine Bestrahlung negativ beeinflusst werden können oder selbst eine Gefahr darstellen.

In der FIG 4 sind noch weitere Schritte gezeigt, die optional vor dem ersten Schritt 22 stattfinden können. So kann die die Röntgenquelle 2 des Röntgengeräts 1 in einem fünften Schritt 20 aktiviert werden, um Röntgenstrahlung auszusenden, welche dann das Untersuchungsobjekt durchstrahlt und dann auf dem Röntgendetektor auftrifft. Zeitnah mit der Aktivierung der Röntgenquelle 2 wird in einem sechsten Schritt 21 ein Triggersignal an das Überwachungssystem übertragen (z.B. kabellos oder mittels einer Kabelverbindung), wodurch die Überwachungseinheit in dem ersten Schritt 22 aktiviert wird.

Durch die Erfindung wird das medizinische Personal insbesondere bei operativen oder interventionellen Eingriffen unter Röntgenüberwachung schnell und aufwandsarm auf die Gefahren durch direkte (und indirekte) Röntgenstrahlung hingewiesen und kann so dauerhaft gesundheitliche Risiken senken.

Die Erfindung lässt sich in folgender Weise kurz zusammenfassen: Verfahren zur Überwachung der Strahlenexposition von medizinischem Personal während einer Röntgenuntersuchung eines Untersuchungsobjekts mit einem Röntgengerät mit den folgenden Schritten: Aktivierung einer Überwachungseinheit, wobei die Überwachungseinheit ein erstes dreidimensionales Volumen, welches einen von dem Röntgenstrahl direkt bestrahlten Bereich umfasst, kontinuierlich nach Objekten abscannt, Im Falle einer Detektion eines Objekts automatische Auswertung, ob es sich bei dem Objekt um einen menschlichen Körperteil handelt, welcher nicht dem Untersuchungsobjekt entspricht, und Ausgabe eines Signals oder einer Anzeige, wenn ein menschlicher Körperteil innerhalb des ersten dreidimensionalen Volumens ermittelt wird, welches nicht dem Untersuchungsobjekt entspricht.

## Patentansprüche

1. Verfahren zur Überwachung der Strahlenexposition von medizinischem Personal (5) während einer Röntgenuntersuchung eines Untersuchungsobjekts (6) mit einem Röntgengerät (1) mit den folgenden Schritten:
• Aktivierung einer Überwachungseinheit (4), wobei die Überwachungseinheit ein erstes dreidimensionales Volumen (8), welches einen von dem Röntgenstrahl direkt bestrahlbaren und/oder bestrahlten Bereich umfasst, kontinuierlich nach Objekten abscannt, wobei die Aktivierung der Überwachungseinheit getriggert wird durch die Aktivierung einer einen Röntgenstrahl aussendenden Röntgenquelle (2),
• Im Falle einer Detektion eines Objekts automatische Auswertung, ob es sich bei dem Objekt um einen menschlichen Körperteil handelt, welcher nicht dem Untersuchungsobjekt (6) entspricht, und
• Ausgabe eines Signals oder einer Anzeige, wenn ein menschlicher Körperteil innerhalb des dreidimensionalen Volumens (8) ermittelt wird, welches nicht dem Untersuchungsobjekt (6) entspricht.

2. Verfahren nach Anspruch 1, wobei das erste dreidimensionale Volumen (8) abhängig von dem Strahlengang des Röntgenstrahls insbesondere automatisch vorgegeben wird.

3. Verfahren nach einem der vorangehenden Ansprüche, wobei die Überwachungseinheit von zumindest einer 3D-Kamera (4) oder einem Infrarotgerät oder einer Terahertzkamera gebildet wird.

4. Verfahren nach einem der vorangehenden Ansprüche, wobei die Auswertung von einem Computervisionsverfahren durchgeführt wird.

5. Verfahren nach einem der vorangehenden Ansprüche, wobei Informationen über den von dem Röntgenstrahl bestrahlten Bereich kontinuierlich erneuert und das erste dreidimensionale Volumen (8) entsprechend angepasst wird.

6. Verfahren nach einem der vorangehenden Ansprüche, wobei für die automatische Auswertung neuronale Netzwerke verwendet werden.

7. Verfahren nach einem der vorangehenden Ansprüche, wobei die Überwachungseinheit zusätzlich ein zweites dreidimensionales Volumen, welches einen von Streustrahlung getroffenen Bereich umfasst, nach Objekten abscannt und automatisch auswertet, ob es sich bei dem Objekt um einen menschlichen Körperteil handelt, welcher nicht dem Untersuchungsobjekt (6) entspricht, und ein Signal oder eine Anzeige ausgegeben wird, wenn ein menschlicher Körperteil innerhalb des zweiten dreidimensionalen Volumens ermittelt wird, welches nicht dem Untersuchungsobjekt (6) entspricht.

8. Verfahren nach einem der vorangehenden Ansprüche, wobei im Fall einer Ermittlung eines menschlichen Körperteils innerhalb des ersten dreidimensionalen Volumens (8), welcher nicht dem Untersuchungsobjekt (6) entspricht, eine Anpassung der Kollimation des Röntgenstrahles der Röntgenquelle (2) durchgeführt wird.

9. Verfahren nach einem der vorangehenden Ansprüche, wobei die Kollimation den von dem Röntgenstrahl bestrahlten Bereich derart ändert, dass der menschliche Körperteil nicht mehr bestrahlt wird.

10. Verfahren nach einem der vorangehenden Ansprüche, wobei das Signal von einem optischen oder akustischen oder haptischen Signal gebildet wird.

11. Verfahren nach einem der vorangehenden Ansprüche, wobei der ermittelte menschliche Körperteil optisch hervorgehoben, insbesondere beleuchtet, wird.

12. Verfahren nach einem der vorangehenden Ansprüche, wobei im Falle einer Detektion eines Objektes zusätzlich eine Auswertung nach weiteren vorbestimmten Gegenständen erfolgt und bei Ermittlung eines solchen Gegenstandes im Strahlengang ein weiteres, sich von dem Signal oder der Anzeige für ein Körperteil unterscheidendes Signal oder eine weitere Anzeige ausgegeben wird.

13. Überwachungssystem zur Durchführung eines Verfahrens nach einem der Ansprüche 1 bis 12, welches einem von einer Systemsteuerung 87) angesteuerten Röntgengerät (1) mit einer zur Aussendung eines Röntgenstrahls ausgebildeten Röntgenquelle (2) zugeordnet ist, aufweisend eine von der Systemsteuerung (2) triggerbare Überwachungseinheit (4), ausgebildet zum kontinuierlichen Abscannen eines ersten dreidimensionalen Volumens (8) nach Objekten, eine Auswerteeinheit (9), ausgebildet zur Auswertung von mittels der Überwachungseinheit (4) detektierten Objekten dahingehend, ob es sich bei dem Objekt um einen menschlichen Körperteil handelt, welcher nicht dem Untersuchungsobjekt (6) entspricht, und eine Ausgabeeinheit (12) zur Ausgabe eines Signals oder einer Anzeige, wobei das Überwachungssystem so ausgebildet ist, dass die Aktivierung der Überwachungseinheit getriggert wird durch die Aktivierung einer einen Röntgenstrahl aussendenden Röntgenquelle (2).

## Claims

1. Method for monitoring the exposure to radiation of medical personnel (5) during an X-ray examination of an examination object (6) with an X-ray apparatus (1), having the following steps:
• activation of a monitoring unit (4), wherein the monitoring unit continuously scans a first three-dimensional volume (8), which comprises a region that can be directly irradiated and/or is irradiated by the X-ray beam, for objects, wherein activation of the monitoring unit is triggered by activation of an X-ray source (2) that emits an X-ray beam,
• in the event of detection of an object, automatic evaluation as to whether the object is a human body part which does not correspond to the examination object (6), and
• outputting a signal or a display if a human body part is determined inside the three-dimensional volume (8) which does not correspond to the examination object (6).

2. Method according to claim 1, wherein the first three-dimensional volume (8) is in particular automatically specified as a function of the beam path of the X-ray.

3. Method according to one of the preceding claims, wherein the monitoring unit is formed by at least one 3D camera (4) or an infrared device or a terahertz camera.

4. Method according to one of the preceding claims, wherein the evaluation is carried out by a computer vision method.

5. Method according to one of the preceding claims, wherein information about the region irradiated by the X-ray beam is continuously renewed and the first three-dimensional volume (8) is adjusted accordingly.

6. Method according to one of the preceding claims, wherein neural networks are used for the automatic evaluation.

7. Method according to one of the preceding claims, wherein the monitoring unit also scans a second three-dimensional volume, which comprises a region affected by scatter radiation, for objects and automatically evaluates whether the object is a human body part which does not correspond to the examination object (6), and a signal or a display is output if a human body part is determined inside the second three-dimensional volume which does not correspond to the examination object (6).

8. Method according to one of the preceding claims, wherein in the event of determination of a human body part inside the first three-dimensional volume (8) which does not correspond to the examination object (6), an adjustment of the collimation of the X-ray of the X-ray source (2) is carried out.

9. Method according to one of the preceding claims, wherein the collimation changes the region irradiated by the X-ray beam in such a way that the human body part is no longer irradiated.

10. Method according to one of the preceding claims, wherein the signal is formed by an optical or acoustic or haptic signal.

11. Method according to one of the preceding claims, wherein the determined human body part is optically highlighted, in particular illuminated.

12. Method according to one of the preceding claims, wherein in the event of detection of an object, an evaluation of further predetermined articles is made and when an article of this kind is determined in the beam path, a further signal, different from the signal or the display for a body part, or a further display is output.

13. Monitoring system for carrying out a method according to one of claims 1 to 12, which is associated with an X-ray apparatus (1) controlled by a system controller 87), with an X-ray source (2) designed for emitting an X-ray, having a monitoring unit (4) that can be triggered by the system controller (2), designed for continuous scanning of a first three-dimensional volume (8) for objects; an evaluation unit (9) designed for evaluation of objects detected by means of the monitoring unit (4) as to whether the object is a human body part which does not correspond to the examination object (6); and an output unit (12) for outputting a signal or a display, wherein the monitoring system is embodied such that activation of the monitoring unit is triggered by activation of an X-ray source (2) that emits an X-ray beam.

## Revendications

1. Procédé de contrôle de l'exposition aux rayonnements du personnel (5) médical pendant un examen aux rayons X d'un objet (6) à examiner par un appareil (1) à rayons X, comprenant les stades suivants :
• activation d'une unité (4) de contrôle, l'unité de contrôle balayant continuellement vers l'objet un premier volume (8) en trois dimensions, qui comprend une partie balayable et/ou balayée directement par le rayonnement X, l'activation de l'unité de contrôle étant déclenchée par l'activation d'une source (2) de rayons X émettant un rayonnement X,
• dans le cas d'une détection d'un objet, évaluation automatique du point de savoir si l'objet est une partie du corps humain, qui ne correspond pas à l'objet (6) à examiner, et
• émission d'un signal ou d'une indication, s'il est déterminé, à l'intérieur du volume (8) en trois dimensions, une partie du corps humain, qui ne correspond pas à l'objet (6) à examiner.

2. Procédé suivant la revendication 1, dans lequel on prescrit, notamment automatiquement le premier volume (8) en trois dimensions en fonction du trajet du rayonnement X.

3. Procédé suivant l'une des revendications précédentes, dans lequel on forme l'unité de contrôle par au moins un appareil (4) photographique en 3D ou un appareil infrarouge ou un appareil photographique térahertz.

4. Procédé suivant l'une des revendications précédentes, dans lequel on effectue l'évaluation par un procédé de vision par ordinateur.

5. Procédé suivant l'une des revendications précédentes, dans lequel on renouvelle continuellement des informations sur la partie exposée aux rayonnements X et on adapte, en conséquence, le premier volume (8) en trois dimensions.

6. Procédé suivant l'une des revendications précédentes, dans lequel on utilise des réseaux neuronaux pour l'évaluation automatique.

7. Procédé suivant l'une des revendications précédentes, dans lequel l'unité de contrôle balaye, en outre, vers l'objet un deuxième volume en trois dimensions, qui comprend une partie concernée par le rayonnement de dispersion et évalue automatiquement si l'objet est une partie du corps humain, qui ne correspond pas à l'objet (6) à examiner et émet un signal ou une indication, s'il est déterminée, à l'intérieur du deuxième volume en trois dimensions, une partie du corps humain, qui ne correspond pas à l'objet (6) à examiner.

8. Procédé suivant l'une des revendications précédentes, dans lequel, dans le cas d'une détermination d'une partie du corps humain à l'intérieur du premier volume (8) en trois dimensions, qui ne correspond pas à l'objet (6) à examiner, on effectue une adaptation de la collimation du rayonnement X de la source (2) de rayons X.

9. Procédé suivant l'une des revendications précédentes, dans lequel on modifie la collimation de la partie exposée au rayonnement X, de manière à ce que la partie du corps humain ne soit plus exposée.

10. Procédé suivant l'une des revendications précédentes, dans lequel on forme le signal d'un signal optique ou acoustique ou haptique.

11. Procédé suivant l'une des revendications précédentes, dans lequel on fait ressortir visuellement, notamment en l'éclairant, la partie du corps humain, qui est déterminée.

12. Procédé suivant l'une des revendications précédentes, dans lequel, dans le cas d'une détection d'un objet, on effectue, en outre, une évaluation vers d'autres objets définis à l'avance et, si l'on détermine un objet de ce genre dans le trajet du faisceau, on émet un autre signal ou une autre indication se distinguant du signal ou de l'indication d'une partie du corps.

13. Système de contrôle pour effectuer un procédé suivant l'une des revendications 1 à 12, qui est associé à un appareil (1) à rayons X, commandé par une commande (87) du système et ayant une source (2) de rayons X, constituée pour émettre un faisceau de rayons X, comportant une unité (4) de contrôle pouvant être déclenchée par la commande (2) du système et constituée pour balayer continuellement un premier volume (8) en trois dimensions vers des objets, une unité (9) d'évaluation, constituée pour évaluer des objet détectés au moyen de l'unité (4) de contrôle, sur le point de savoir si l'objet est une partie du corps humain, qui ne correspond pas à l'objet (6) à examiner, et une unité (12) d'émission pour émettre un signal ou une indication, le système de contrôle étant constitué de manière à ce que l'activation de l'unité de contrôle soit déclenchée par l'activation d'une source (2) de rayons X émettant un faisceau de rayons X.
